# FASCICULE DE BREVET EUROPEEN

(11) **EP 2 246 039 B1**
(45) Date de publication et mention de la délivrance du brevet: **10.08.2016**
(21) Numéro de dépôt: 10161414.7
(22) Date de dépôt: 29.04.2010
(51) Int. Cl.: A61K 8/58, A61Q 5/10

(54) **Procédé de coloration des cheveux comprenant une étape de traitement des cheveux à partir d'un composé organique du silicium**
Haarfärbeverfahren das einen Schritt zur Behandlung des Haares mittels einer organischen Silikon verbindung umfasst
Method for dyeing hair comprising the step of treating the hair with an organic silicon compound

(30) Priorité: 30.04.2009 FR 0952913
(43) Date de publication de la demande: 03.11.2010
(73) Titulaire: L'Oréal, 75008 Paris (FR)
(72) Inventeur: Charrier, Delphine, 75016, Paris (FR); Audousset, Marie-Pascale, 92600, Asnieres (FR); Hercouet, Leïla, 93360, Neuilly Plaisance (FR)
(74) Mandataire: Prevel, Estelle Nicole

(56) Documents cités:
- EP-A2- 1 312 346
- EP-A2- 1 767 187
- EP-A2- 1 767 189
- WO-A1-01/22925
- WO-A1-01/22931
- WO-A2-2006/118942
- FR-A1- 2 783 164

## Description

La présente invention concerne un procédé de coloration des cheveux comprenant une étape mettant en oeuvre un composé organique du silicium.

Les cheveux sont généralement abîmés et fragilisés par l'action des agents atmosphériques extérieurs tels que la lumière et les intempéries, et par des traitements mécaniques ou chimiques tels que le brossage, le peignage, les décolorations, les permanentes et / ou les teintures. Il en résulte que les cheveux sont souvent difficiles à discipliner, en particulier ils sont difficiles à démêler et à coiffer, et les chevelures, même abondantes, conservent en particulier difficilement une coiffure de bon aspect en raison du fait que les cheveux manquent de vigueur, de volume et de nervosité.

Cette dégradation des propriétés des cheveux est par ailleurs accrue par les traitements de coloration permanente des cheveux, qui consistent à appliquer sur les cheveux un ou plusieurs précurseurs de colorant tels que des bases d'oxydation et des coupleurs et un agent oxydant. Ces précurseurs sous l'action de l'agent oxydant vont former dans le cheveu une ou plusieurs espèces colorées.

Parallèlement, on a constaté que les consommateurs sont de plus en plus à la recherche de compositions qui permettent non seulement de colorer les cheveux de manière satisfaisante mais également de procurer des effets coiffants satisfaisants.

En particulier, les personnes ayant des cheveux fins ou bouclés sont généralement à la recherche d'un effet coiffant apportant de la masse, du corps et du volume aux cheveux fins et du dessin aux boucles des cheveux frisés.

Il est usuel d'appliquer après coloration des produits de conditionnement des cheveux qui permettent d'apporter du démêlage et d'améliorer le toucher, mais ces produits n'apportent pas suffisamment d'effets coiffants notamment en terme de corps, de masse ou de volume.
Les documents EP 1767189 et EP1767187 proposent des compositions à effet coiffant puissant et résistant comprenant des composés organiques du silicium en particulier le N-(2-aminoéthyl)aminométhylphénéthyltriméthoxysilane et le bis[3 (triéthoxysilyl)propyl]amine.
Les documents FR2783164 et WO 01/22925 décrivent des compositions aqueuses pour le traitement ou le soin de cheveux comprenant des composés organiques du silicium et notamment le 3-Aminopropyltriethoxysilane, visant à conférer aux cheveux un effet coiffant de longue durée et un toucher agréable.
Il existe donc un réel besoin de mettre en oeuvre un procédé de coloration permanente des cheveux avec des précurseurs de colorant tels que des bases d'oxydation et des coupleurs, qui ne présente pas les inconvénients décrits ci-dessus, c'est-à-dire qui permet de conduire à des effets coiffants suffisants notamment en terme de corps, de masse ou de volume avec de plus, une rémanence aux shampoings et aux agressions extérieures de ces effets avec de bonnes propriétés cosmétiques, et tout ceci quel que soit la sensibilisation des cheveux traités.

Le but de la présente invention est donc de fournir un procédé de coloration des cheveux qui permet de colorer de façon satisfaisante les cheveux avec au final un effet coiffant suffisant et durable.
Ce but est atteint avec la présente invention qui a pour objet un procédé de coloration des cheveux qui comprend dans l'ordre une étape de coloration par application d'une composition comprenant un ou plusieurs précurseurs de colorant, une étape de rinçage et une étape de post-traitement des cheveux par application d'une composition aqueuse comprenant au moins 30 % en poids d'eau, au moins 20 % en poids d'un ou plusieurs composés organiques du silicium choisis parmi les composés de formule (III) : 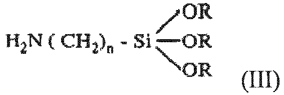 dans laquelle les radicaux R, identiques ou différents, sont choisis parmi les radicaux alkyle en C₁-C₆ et n est un nombre entier de 1 à 6, de préférence de 2 à 4.

Le procédé de la présente invention permet d'obtenir une coloration des cheveux avec des propriétés tinctoriales très satisfaisantes, notamment en terme d'intensité, de chromaticité, de sélectivité et une bonne résistance de cette couleur aux agents extérieurs tels que la résistance aux shampoings, à la sueur, aux intempéries tout en conférant aux cheveux du coiffant, de la masse et du corps

Le ou les composés organiques du silicium utilisés dans la composition selon invention sont des organosilanes choisis parmi les composés de formule (III) : 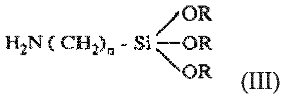 dans laquelle les radicaux R, identiques ou différents, sont choisis parmi les radicaux alkyle en C₁-C₆, de préférence en C₁-C₂, et n est un nombre entier de 1 à 6, de préférence de 2 à 4.

Ces organosilanes sont de préférence solubles dans l'eau et encore plus préférentiellement solubles à la concentration de 2%, mieux à la concentration de 5% et encore mieux à la concentration de 10% en poids dans l'eau à la température de 25°C±5°C et à la pression atmosphérique.Par soluble, on entend la formation d'une phase macroscopique unique.

De façon particulièrement préférée, le composé organique du silicium présent dans la composition selon l'invention est le 3-aminopropyl triéthoxysilane.

Le ou les composés organiques du silicium peuvent être présents dans la composition de préférence dans une teneur allant de de 20 à 65%, encore plus préférentiellement de 30 à 60%, mieux de 40 à 50% en poids, par rapport au poids total de la composition.

Selon un mode de réalisation particulier l'eau est présente dans cette composition dans une teneur allant de de 30 à 78 %, préférentiellement de 40 à 70%, mieux de 45 à 60% en poids, par rapport au poids total de la composition.

Le ou les composés organiques de silicium peuvent être partiellement neutralisés au moyen d'un agent de neutralisation ou régulateur de pH, de telle sorte que la neutralisation atteigne 1/1000 à 99/100 et mieux de 0,2/100 à 70/100. De préférence encore, la neutralisation est de 0,2/100 à 60/100.

Les agents régulateurs de pH peuvent être tous les acides ou mélanges d'acides cosmétiquement acceptables et solubles dans le milieu de la composition. Parmi les acides utilisables, on peut citer l'acide chlorhydrique, l'acide phosphorique, l'acide sulfonique et les acides organiques. La composition utilisée selon l'invention peut également contenir un ou plusieurs autres acides organiques.

Les acides organiques sont généralement choisis parmi les acides comportant une ou plusieurs fonctions acide carboxylique, sulfonique, phosphonique ou phosphorique. Ils peuvent contenir d'autres fonctions chimiques, en particulier des fonctions hydroxy ou amino. Ils peuvent être saturés ou insaturés. On peut citer en particulier l'acide acétique, l'acide propanoïque, l'acide butanoïque, l'acide lactique, l'acide glycolique, l'acide ascorbique, l'acide maléique, l'acide phtalique, l'acide succinique, la taurine, l'acide tartrique, l'acide gluconique, l'acide glucuronique et l'acide citrique. Les acides organiques préférés sont l'acide lactique, l'acide acétique, l'acide citrique.

La composition de post traitement comprenant le composé organique du silicium présente généralement un pH compris entre 2 et 13, de préférence entre 4 et 11. Plus préférentiellement, le pH de cette composition obtenue avec l'agent régulateur de pH est compris entre 6 et 11, encore plus préférentiellement, le pH est compris entre 8 et 10.

La composition contenant le ou les composés organiques du silicium peut en plus contenir un ou plusieurs épaississants. Les agents épaississants peuvent être choisis parmi les amides d'acides gras (diéthanol- ou monoéthanol-amide de coprah, monoéthanolamide d'acide alkyl éther carboxylique oxyéthyléné), les épaississants cellulosiques (hydroxyéthycellulose, hydroxypropylcellulose, carboxyméthylcellulose), la gomme de guar et ses dérivés (hydroxypropylguar), les gommes d'origine microbienne (gomme de xanthane, gomme de scléroglucane), les homopolymères réticulés d'acide acrylique ou d'acide acrylamidopropanesulfonique.

La composition de coloration des cheveux comprend un ou plusieurs précurseurs de colorant pouvant être choisi(s) parmi les bases d'oxydation et les coupleurs.

La (les) base(s) d'oxydation est (sont) choisie(s) parmi celles classiquement connues en teinture d'oxydation, et parmi lesquelles on peut notamment citer les ortho- et para-phénylènediamines, les bases doubles, les ortho- et para- aminophénols, les bases hétérocycliques ainsi que les sels d'addition de ces composés avec un acide.

Ces bases d'oxydation peuvent être en particulier cationiques.

Les para-phénylènediamines peuvent notamment être choisies parmi les composés de formule (IV) suivante et leurs sels d'addition avec un acide : 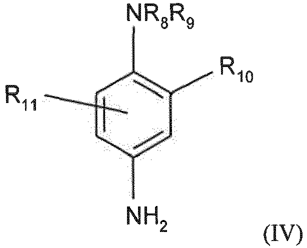 dans laquelle :
▪ R₈ représente un atome d'hydrogène, un radical alkyle en C₁-C₄, monohydroxyalkyle en C₁-C₄, polyhydroxyalkyle en C₂-C₄, alcoxy(C₁-C₄)alkyle(C₁-C₄), alkyle en C₁-C₄ substitué par un groupement azoté, phényle ou 4'-aminophényle ;
▪ R₉ représente un atome d'hydrogène, un radical alkyle en C₁-C₄, monohydroxyalkyle en C₁-C₄ ou polyhydroxyalkyle en C₂-C₄, alcoxy(C₁-C₄)alkyle(C₁-C₄) ou alkyle en C₁-C₄ substitué par un groupement azoté ;
▪ R₈ et R₉ peuvent également former avec l'atome d'azote qui les porte un hétérocycle azoté à 5 ou 6 chaînons éventuellement substitué par un ou plusieurs groupements alkyle, hydroxy ou uréido ;
▪ R₁₀ représente un atome d'hydrogène, un atome d'halogène tel qu'un atome de chlore, un radical alkyle en C₁-C₄, sulfo, carboxy, monohydroxyalkyle en C₁-C₄ ou hydroxyalcoxy en C₁-C₄, acétylaminoalcoxy en C₁-C₄, mésylaminoalcoxy en C₁-C₄ ou carbamoylaminoalcoxy en C₁-C₄ ;
▪ R₁₁ représente un atome d'hydrogène, d'halogène ou un radical alkyle en C₁-C₄.

Parmi les groupements azotés de la formule (IV) ci-dessus, on peut citer notamment les radicaux amino, monoalkyl(C₁-C₄)amino, dialkyl(C₁-C₄)amino, trialkyl(C₁-C₄)amino, monohydroxyalkyl(C₁-C₄)amino, imidazolinium et ammonium.

Parmi les para-phénylènediamines de formule (IV) ci-dessus, on peut plus particulièrement citer la para-phénylènediamine, la para-toluylènediamine, la 2-chloro-para-phénylènediamine, la 2,3-diméthyl-para-phénylènediamine, la 2,6-diméthyl-para-phénylènediamine, la 2,6-diéthyl-para-phénylènediamine, la 2,5-diméthyl-para-phénylènediamine, la N,N-diméthyl-para-phénylènediamine, la N,N-diéthyl-para-phénylènediamine, la N,N-dipropyl-para-phénylènediamine, la 4-amino-N,N-diéthyl-3-méthyl-aniline, la N,N-bis-(β-hydroxyéthyl)-para-phénylènediamine, la 4-N,N-bis-(β-hydroxyéthyl)amino-2-méthyl-aniline, la 4-N,N-bis-(β-hydroxyéthyl)-amino-2-chloro-aniline, la 2-(β-hydroxyéthyl-para-phénylènediamine, la 2-fluoro-para-phénylènediamine, la 2-isopropyl-para-phénylènediamine, la N-(β-hydroxypropyl)-para-phénylènediamine, la 2-hydroxyméthyl-para-phénylènediamine, la N,N-diméthyl-3-méthyl-para-phénylènediamine, la N,N-(éthyl,β-hydroxyéthyl)-para-phénylènediamine, la N-(β,γ-dihydroxypropyl)-para-phénylènediamine, la N-(4'-aminophényl)-para-phénylènediamine, la N-phényl-para-phénylènediamine, la 2-β-hydroxyéthyloxy-para-phénylènediamine, la 2-β-acétylaminoéthyloxy-para-phénylènediamine, la N-(β-méthoxyéthyl)-para-phénylènediamine, la 2-méthyl-1-N-β-hydroxyéthyl-para-phénylénediamine, et leurs sels d'addition avec un acide.

Parmi les para-phénylènediamines de formule (IV) ci-dessus, on préfère tout particulièrement la para-phénylènediamine, la para-toluylènediamine, la 2-isopropyl-para-phénylènediamine, la 2-β-hydroxyéthyl-para-phénylènediamine, la 2,6-diméthyl-para-phénylène-diamine, la 2,6-diéthyl-para-phénylènediamine, la 2,3-diméthyl-para-phénylènediamine, la N,N-bis-(β-hydroxyéthyl)-para-phénylènediamine, la 2-chloro-para-phénylènediamine, et leurs sels d'addition avec un acide.

On utilisera tout particulièrement la para-phénylènediamine, la para-toluylènediamine, la N,N-bis-(β-hydroxyéthyl)-para-phénylènediamine, et leurs sels d'addition avec un acide.

Selon l'invention, on entend par bases doubles les composés comportant au moins deux noyaux aromatiques sur lesquels sont portés des groupements amino et/ou hydroxyle.

Parmi les bases doubles, on peut notamment citer les composés répondant à la formule (V) suivante et leurs sels d'addition avec un acide : 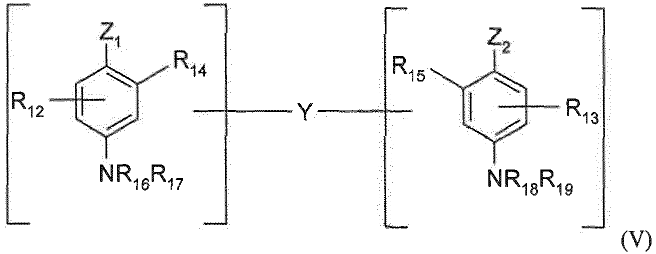 dans laquelle :
▪ Z₁ et Z₂, identiques ou différents, représentent un radical hydroxyle ou-NH₂ pouvant être substitué par un radical alkyle en C₁-C₄ ou par un bras de liaison Y ;
▪ le bras de liaison Y représente une chaîne alkylène comportant de 1 à 14 atomes de carbone, linéaire ou ramifiée pouvant être interrompue ou terminée par un ou plusieurs groupements azotés et/ou par un ou plusieurs hétéroatomes tels que des atomes d'oxygène, de soufre ou d'azote, et éventuellement substituée par un ou plusieurs radicaux hydroxyle ou alcoxy en C₁-C₆ ;
▪ R₁₂ et R₁₃ représentent un atome d'hydrogène ou d'halogène, un radical alkyle en C₁-C₄, monohydroxyalkyle en C₁-C₄, polyhydroxyalkyle en C₂-C₄, aminoalkyle en C₁-C₄ ou un bras de liaison Y ;
▪ R₁₄, R₁₅, R₁₆, R₁₇, R₁₈ et R₁₉, identiques ou différents, représentent un atome d'hydrogène, un bras de liaison Y ou un radical alkyle en C₁-C₄ ;
▪ étant entendu que les composés de formule (V) ne comportent qu'un seul bras de liaison Y par molécule.

Parmi les groupements azotés de la formule (V) ci-dessus, on peut citer notamment les radicaux amino, monoalkyl(C₁-C₄)amino, dialkyl(C₁-C₄)amino, trialkyl(C₁-C₄)amino, monohydroxyalkyl(C₁-C₄)amino, imidazolinium et ammonium.

Parmi les bases doubles de formules (V) ci-dessus, on peut plus particulièrement citer le N,N'-bis-(β-hydroxyéthyl)-N,N'-bis-(4'-aminophényl)-1,3-diamino-propanol, la N,N'-bis-(β-hydroxyéthyl)-N,N'-bis-(4'-aminophényl)-éthylènediamine, la N,N'-bis-(4-aminophényl)-tétraméthylènediamine, la N,N'-bis-(β-hydroxyéthyl)-N,N'-bis-(4-aminophényl)-tétraméthylènediamine, la N,N'-bis-(4-méthyl-aminophényl)-tétraméthylènediamine, la N,N'-bis-(éthyl)-N,N'-bis-(4'-amino-3'-méthylphényl)-éthylènediamine, le 1,8-bis-(2,5-diaminophénoxy)-3,5-dioxaoctane, et leurs sels d'addition avec un acide.

Parmi ces bases doubles de formule (V), le N,N'-bis-(β-hydroxyéthyl)-N,N'-bis-(4'-aminophényl)-1,3-diamino-propanol, le 1,8-bis-(2,5-diaminophénoxy)-3,5-dioxaoctane ou l'un de leurs sels d'addition avec un acide sont particulièrement préférés.

Les para-aminophénols peuvent notamment être choisis parmi les composés répondant à la formule (VI) suivante et leurs sels d'addition avec un acide : 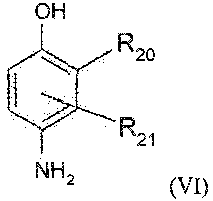 dans laquelle :
▪ R₂₀ représente un atome d'hydrogène, un atome d'halogène tel que le fluor, un radical alkyle en C₁-C₄, monohydroxyalkyle en C₁-C₄, alcoxy(C₁-C₄)alkyle(C₁-C₄) ou aminoalkyle en C₁-C₄, ou hydroxyalkyl(C₁-C₄)aminoalkyle en C₁-C₄ ;
▪ R₂₁ représente un atome d'hydrogène ou un atome d'halogène tel que le fluor, un radical alkyle en C₁-C₄, monohydroxyalkyle en C₁-C₄, polyhydroxyalkyle en C₂-C₄, aminoalkyle en C₁-C₄, cyanoalkyle en C₁-C₄ ou alcoxy(C₁-C₄)alkyle(C₁-C₄).

Parmi les para-aminophénols de formule (VI) ci-dessus, on peut plus particulièrement citer le para-aminophénol, le 4-amino-3-méthyl-phénol, le 4-amino-3-fluoro-phénol, le 4-amino-3-hydroxyméthyl-phénol, le 4-amino-2-méthyl-phénol, le 4-amino-2-hydroxyméthyl-phénol, le 4-amino-2-méthoxyméthyl-phénol, le 4-amino-2-aminométhyl-phénol, le 4-amino-2-(β-hydroxyéthyl-aminométhyl)-phénol, et leurs sels d'addition avec un acide.

Le para-aminophénol et le 4-amino-3-méthyl-phénol sont encore plus préférés.

Les ortho-aminophénols sont notamment choisis parmi le 2-amino-phénol, le 2-amino-1-hydroxy-5-méthyl-benzène, le 2-amino-1-hydroxy-6-méthyl-benzène, le 5-acétamido-2-amino-phénol, et leurs sels d'addition avec un acide.

Parmi les bases hétérocycliques, on peut plus particulièrement citer les dérivés pyridiniques, les dérivés pyrimidiniques, les dérivés pyrazoliques, et leurs sels d'addition avec un acide.

Parmi les dérivés pyridiniques, on peut plus particulièrement citer les composés décrits par exemple dans les brevets GB 1 026 978 et GB 1 153 196, comme la 2,5-diamino-pyridine, la 2-(4-méthoxyphényl)amino-3-amino-pyridine, la 2,3-diamino-6-méthoxy-pyridine, la 2-(β-méthoxyéthyl)amino-3-amino-6-méthoxy pyridine, la 3,4-diamino-pyridine, et leurs sels d'addition avec un acide.

Parmi les dérivés pyrimidiniques, on peut plus particulièrement citer les composés décrits par exemple dans les brevets allemand DE 2 359 399 ou japonais JP 88-169 571 et JP 91-10659 ou demandes de brevet WO 96/15765, comme la 2,4,5,6-tétra-aminopyrimidine, la 4-hydroxy-2,5,6-triaminopyrimidine, la 2-hydroxy-4,5,6-triaminopyrimidine, la 2,4-dihydroxy-5,6-diaminopyrimidine, la 2,5,6-triaminopyrimidine, et les dérivés pyrazolo-pyrimidiniques tels ceux mentionnés dans la demande de brevet FR-A-2 750 048 et parmi lesquels on peut citer la pyrazolo-[1,5-a]-pyrimidine-3,7-diamine ; la 2,5-diméthyl-pyrazolo-[1,5-a]-pyrimidine-3,7-diamine ; la pyrazolo-[1,5-a]-pyrimidine-3,5-diamine ; la 2,7-diméthyl-pyrazolo-[1,5-a]-pyrimidine-3,5-diamine ; le 3-amino-pyrazolo-[1,5-a]-pyrimidin-7-ol ; le 3-amino-pyrazolo-[1,5-a]-pyrimidin-5-ol ; le 2-(3-amino pyrazolo-[1,5-a]-pyrimidin-7-ylamino)-éthanol; le 2-(7-amino-pyrazolo-[1,5-a]-pyrimidin-3-ylamino)-éthanol ; le 2-[(3-amino-pyrazolo[1,5-a]pyrimidin-7-yl)-(2-hydroxy-éthyl)-amino]-éthanol ; le 2-[(7-amino-pyrazolo[1,5-a]pyrimidin-3-yl)-(2-hydroxy-éthyl)-amino]-éthanol ; la 5,6-diméthyl-pyrazolo-[1,5-a]-pyrimidine-3,7-diamine ; la 2,6-diméthyl-pyrazolo-[1,5-a]-pyrimidine-3,7-diamine ; la 2,5,N7,N7-tétraméthyl-pyrazolo-[1,5-a]-pyrimidine-3,7-diamine ; la 3-amino-5-méthyl-7-imidazolylpropylamino-pyrazolo-[1,5-a]-pyrimidine ; leurs sels d'addition et leurs formes tautomères, lorsqu'il existe un équilibre tautomérique.

Parmi les dérivés pyrazoliques, on peut plus particulièrement citer les composés décrits dans les brevets DE 3 843 892, DE 4 133 957 et demandes de brevet WO 94/08969, WO 94/08970, FR-A-2 733 749 et DE 195 43 988 comme les 4,5-diaminopyrazoles tels que par exemple le 4,5-diamino-1-méthyl-pyrazole, le 4,5-diamino-1-(4'-chlorobenzyl)-pyrazole, le 4,5-diamino-1,3-diméthyl-pyrazole, le 4,5-diamino-3-méthyl-1-phényl-pyrazole, le 4,5-diamino-1-méthyl-3-phényl-pyrazole, le 1-benzyl-4,5-diamino-3-méthyl-pyrazole, le 4,5-diamino-3-tert-butyl-1-méthyl-pyrazole, le 4,5-diamino-1-tert-butyl-3-méthyl-pyrazole, le 4,5-diamino-1-(β-hydroxyéthyl)-3-méthyl-pyrazole, le 4,5-diamino-1-(β-hydroxyéthyl)-pyrazole, le 4,5-diamino-1-éthyl-3-méthyl-pyrazole, le 4,5-diamino-1-éthyl-3-(4'-méthoxyphényl)-pyrazole, le 4,5-diamino-1-éthyl-3-hydroxyméthyl-pyrazole, le 4,5-diamino-3-hydroxyméthyl-1-méthyl-pyrazole, le 4,5-diamino-3-hydroxyméthyl-1-isopropyl-pyrazole, le 4,5-diamino-3-méthyl-1-isopropyl-pyrazole et le 4-amino-5-(2'-aminoéthyl)amino-1,3-diméthyl-pyrazole ; le 3,4-diamino-pyrazole ; le 4-amino-1,3-diméthyl-5-hydrazino-pyrazole ; les 3,4,5-triaminopyrazoles tels que par exemple le 3,4,5-triamino-pyrazole, le 1-méthyl-3,4,5-triamino-pyrazole, le 3,5-diamino-1-méthyl-4-méthylamino-pyrazole, le 3,5-diamino-4-(β-hydroxyéthyl)amino-1-méthyl-pyrazole ; et leurs sels d'addition avec un acide.

De préférence, on utilisera un 4,5-diaminopyrazole et encore plus préférentiellement le 4,5-diamino-1-(β-hydroxyéthyl)-pyrazole et/ou l'un des ses sels.

A titre de dérivés pyrazoliques, on peut également citer les diamino N,N-dihydropyrazolopyrazolones et notamment celles décrites dans la demande FR-A-2 886 136 telles que les composés suivants et leurs sels d'addition : 2,3-diamino-6,7-dihydro-1H,5H-pyrazolo[1,2-a]pyrazol-1-one, 2-amino-3-éthylamino-6,7-dihydro-1H,5H-pyrazolo[1,2-a]pyrazol-1-one, 2-amino-3-isopropylamino-6,7-dihydro-1H,5H-pyrazolo[1,2-a]pyrazol-1-one, 2-amino-3-(pyrrolidin-1-yl)-6,7-dihydro-1H,5H-pyrazolo[1,2-a]pyrazol-1-one, 4,5-diamino-1,2-diméthyl-1,2-dihydro-pyrazol-3-one, 4,5-diamino-1,2-diéthyl-1,2-dihydro-pyrazol-3-one, 4,5-diamino-1,2-di-(2-hydroxyéthyl)-1,2-dihydro-pyrazol-3-one, 2-amino-3-(2-hydroxyéthyl)amino-6,7-dihydro-1H,5H-pyrazolo[1,2-a]pyrazol-1-one, 2-amino-3-diméthylamino-6,7-dihydro-1H,5H-pyrazolo[1,2-a]pyrazol-1-one, 2,3-diamino-5,6,7,8-tétrahydro-1H,6H-pyridazino[1,2-a]pyrazol-1-one, 4-amino-1,2-diéthyl-5-(pyrrolidin-1-yl)-1,2-dihydro-pyrazol-3-one, 4-amino-5-(3-diméthylamino-pyrrolidin-1-yl)-1,2-diéthyl-1,2-dihydro-pyrazol-3 -one, 2,3-diamino-6-hydroxy-6,7-dihydro-1H,5H-pyrazolo[1,2-a]pyrazol-1-one.

On préférera utiliser la 2,3-diamino-6,7-dihydro-1H,5H-pyrazolo[1,2-a]pyrazol-1-one et/ou un de ses sels d'addition.

A titre de bases hétérocycliques, on utilisera préférentiellement le 4,5-diamino-1-(β-hydroxyéthyl)pyrazole et/ou la 2,3-diamino-6,7-dihydro-1H,5H-pyrazolo[1,2-a]pyrazol-1-one et/ou un de leurs sels d'addition.

A titre de bases d'oxydation cationiques, on peut citer par exemple les composés suivants : les para-phénylènediamines telles que notamment décrites dans les demandes de brevets FR-A-2 766 177 et FR-A-2 766 178, les para-aminophénols tels que décrits par exemple dans les demandes de brevet FR-A-2 766 177 et FR-A-2 766 178, les ortho-phénylènediamines telles que décrites par exemple dans les demandes de brevet FR-A-2 782 718, FR-A-2 782 716 et FR-A-2 782 719, des ortho-aminophénols ou des bases doubles cationiques telles que des dérivés de type bis(aminophényl)alkylènediamine décrites dans les demandes de brevet FR-A-2 766 179, ainsi que les bases hétérocycliques cationiques, ces composés portant au moins un atome d'azote quaternaire.

De préférence, les bases d'oxydation cationiques sont des para-phénylènediamines cationiques.

De manière avantageuse, une variante consiste à mettre en oeuvre des bases d'oxydation cationiques de structure para-phénylènediamine, dont au moins une des fonctions amine est une amine tertiaire porteuse d'un noyau pyrrolidinique, la molécule possédant au moins un atome d'azote quaternisé. De telles bases sont, par exemple, décrites dans le document EP-A-1 348 695.

La composition de coloration comprend de préférence une quantité totale de base(s) d'oxydation allant de 0,0005 à 12 % en poids par rapport au poids total de la composition. De préférence, elle comprend une quantité totale de base(s) d'oxydation allant de 0,005 à 8 % en poids, et mieux encore de 0,05 à 5 % en poids, par rapport au poids total de ladite composition.

Le(s) coupleur(s) est celui (sont ceux) classiquement utilisé(s) dans les compositions de teinture d'oxydation, c'est-à-dire les méta-aminophénols, les méta-phénylènediamines, les métadiphénols, les naphtols et les coupleurs hétérocycliques tels que, par exemple, les dérivés indoliques, les dérivés indoliniques, le sésamol et ses dérivés, les dérivés pyridiniques, les dérivés pyrazolotriazoles, les pyrazolones, les indazoles, les benzimidazoles, les benzothiazoles, les benzoxazoles, les 1,3-benzodioxoles, les quinolines, et les sels d'addition de ces composés avec un acide.

Ces coupleurs sont plus particulièrement choisis parmi le 2,4-diamino 1-(β-hydroxyéthyloxy)-benzène, le 2-méthyl-5-amino-phénol, le 5-N-(β-hydroxyéthyl)amino-2-méthyl-phénol, le 3-amino-phénol, le 1,3-dihydroxy-benzène, le 1,3-dihydroxy-2-méthyl-benzène, le 4-chloro-1,3-dihydroxy-benzène, le 2-amino 4-(β-hydroxyéthylamino)-1-méthoxy-benzène, le 1,3-diamino-benzène, le 1,3-bis-(2,4-diaminophénoxy)-propane, le sésamol, le 1-amino-2-méthoxy-4,5-méthylènedioxy benzène, l'α-naphtol, le 6-hydroxy-indole, le 4-hydroxy-indole, le 4-hydroxy-N-méthyl indole, la 6-hydroxy-indoline, la 2,6-dihydroxy-4-méthyl-pyridine, le 1-H-3-méthyl-pyrazole-5-one, le 1-phényl-3-méthyl-pyrazole-5-one, la 2-amino-3-hydroxypyridine, le 3,6-diméthyl-pyrazolo-[3,2-c]-1,2,4-triazole, le 2,6-diméthyl-pyrazolo-[1,5-b]-1,2,4-triazole et leurs sels d'addition avec un acide.

La composition comprend généralement une quantité totale de coupleur(s) allant de 0,0001 à 15 % en poids par rapport au poids total de la composition. De préférence, elle comprend une quantité totale de coupleur(s) allant de 0,001 à 10 % en poids, et mieux encore de 0,01 à 8 % en poids, par rapport au poids total de la composition.

Les bases d'oxydation et coupleurs peuvent être présents dans les compositions de l'invention, sous forme de sels d'addition, et en particulier sous forme de sels d'addition avec un acide.

Les sels d'addition avec un acide utilisables dans le cadre de l'invention sont, notamment, choisis parmi les chlorhydrates, les bromhydrates, les sulfates, les citrates, les succinates, les tartrates, les lactates, les acétates, les alkylsulfates et les alkylsulfonates.

Lorsque les bases d'oxydation ou les coupleurs contiennent une ou plusieurs fonction(s) acide carboxylique ou sulfonique, des sels d'addition avec une base sont envisageables. Les sels d'addition avec une base utilisables dans le cadre des compositions tinctoriales de l'invention sont alors notamment ceux obtenus avec de la soude, de la potasse, de l'ammoniaque ou des amines.

Selon un mode de réalisation particulier de l'invention, la composition comprend une ou plusieurs base(s) d'oxydation et un ou plusieurs coupleur(s).

Selon un mode de réalisation préféré, la composition de coloration contient en outre un ou plusieurs polymères cationiques dont la densité de charge cationique est supérieure ou égale à 4 milliéquivalents par gramme (meq/g), de préférence supérieure ou égale à 5 milliéquivalents par gramme (meq/g), de préférence allant de 5 à 20 méq/g et plus particulièrement de 5,5 à 10 meq/g.

La densité de charge cationique d'un polymère correspond au nombre de moles de charges cationiques par unité de masse de polymère dans les conditions où celui-ci est totalement ionisé. Elle peut être déterminée par calcul si on connaît la structure du polymère, c'est-à-dire la structure des monomères constituant le polymère et leur proportion molaire ou pondérale. Elle peut être aussi déterminée expérimentalement par la méthode Kjeldahl, généralement à un pH d'environ 7 à température ambiante.

Les polymères cationiques ayant une densité de charge cationique supérieure à 4 méq/g, peuvent être choisis parmi tous ceux déjà connus en soi comme améliorant les propriétés cosmétiques des cheveux traités par des compositions, à savoir notamment ceux décrits dans la demande de brevet EP-A-0 337 354 et dans les demandes de brevets français FR-A-2 270 846, 2 383 660, 2 598 611, 2 470 596 et 2 519 863.

De manière générale, au sens de la présente invention, l'expression "polymère cationique" désigne tout polymère comprenant des groupes cationiques et/ou des groupes ionisables en groupes cationiques..

Les polymères cationiques sont choisis parmi ceux qui comprennent des motifs comportant des groupements amine primaires, secondaires, tertiaires et/ou quaternaires pouvant soit faire partie de la chaîne principale polymère, soit être portés par un substituant latéral directement relié à celle-ci.

Les polymères cationiques utilisés ont généralement une masse molaire moyenne en nombre comprise entre 500 et 5.10⁶ environ, et de préférence comprise entre 10³ et 3.10⁶ environ.

Parmi les polymères cationiques, on peut citer plus particulièrement les polymères du type polyamine, polyaminoamide et polyammonium quaternaire. Ce sont des produits connus.

Parmi ces polymères, on peut citer :
(1) les homopolymères ou copolymères dérivés d'esters ou d'amides acryliques ou méthacryliques et comportant au moins un des motifs de formules suivantes: 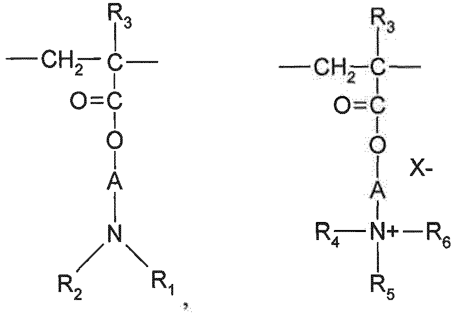 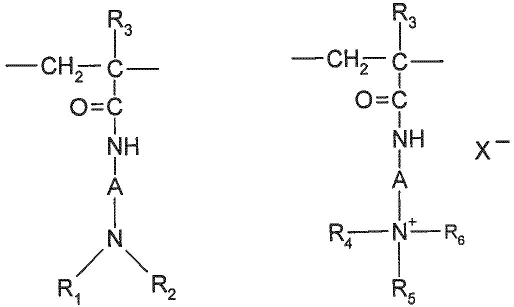 dans lesquelles:
   R₃, identiques ou différents, désignent un atome d'hydrogène ou un radical CH₃;
   A, identiques ou différents, représentent un groupe alkyle, linéaire ou ramifié, de 1 à 6 atomes de carbone, de préférence 2 ou 3 atomes de carbone ou un groupe hydroxyalkyle de 1 à 4 atomes de carbone ;
   R₄, R₅, R₆, identiques ou différents, représentent un groupe alkyle ayant de 1 à 18 atomes de carbone ou un radical benzyle et de préférence un groupe alkyle ayant de 1 à 6 atomes de carbone;
   R₁ et R₂, identiques ou différents, représentent hydrogène ou un groupe alkyle ayant de 1 à 6 atomes de carbone et de préférence méthyle ou éthyle;
   X désigne un anion dérivé d'un acide minéral ou organique en particulier un anion méthosulfate ou un halogénure, notamment chlorure ou bromure.

   Les copolymères de la famille (1) peuvent comprendre en outre un ou plusieurs motifs dérivant de comonomères pouvant être choisis dans la famille des acrylamides, méthacrylamides, diacétones acrylamides, acrylamides et méthacrylamides substitués sur l'azote par des alkyles inférieurs (C1-C4), des acides acryliques ou méthacryliques ou leurs esters, des vinyllactames tels que la vinylpyrrolidone ou le vinylcaprolactame, des esters vinyliques.
   Ainsi, parmi ces copolymères de la famille (1), on peut citer :
   - les copolymères d'acrylamide et de diméthylaminoéthyl méthacrylate quaternisé au sulfate de diméthyle ou avec un halogénure de diméthyle
   - les copolymères d'acrylamide et de chlorure de méthacryloyloxyéthyltriméthyl-ammonium décrit par exemple dans la demande de brevet EP-A-080976
   - le copolymères d'acrylamide et de méthosulfate de méthacryloyloxyéthyl-triméthylammonium,
   - les copolymères vinylpyrrolidone / acrylate ou méthacrylate de dialkylaminoalkyle quaternisés ou non. Ces polymères sont décrits en détail dans les brevets français 2.077.143 et 2.393.573,
   - les terpolymères méthacrylate de diméthyl amino éthyle/ vinylcaprolactame/ vinylpyrrolidone,
   - les copolymères vinylpyrrolidone / méthacrylamidopropyl dimethylamine.
   - les copolymères vinylpyrrolidone / méthacrylamide de diméthylaminopropyle quaternisé,
   - et les polymères réticulés de sels de méthacryloyloxyalkyl(C1-C4) trialkyl(C1-C4)ammonium tels que les polymères obtenus par homopolymérisation du diméthylaminoéthylméthacrylate quaternisé par le chlorure de méthyle, ou par copolymérisation de l'acrylamide avec le diméthylaminoéthylméthacrylate quaternisé par le chlorure de méthyle, l'homo ou la copolymérisation étant suivie d'une réticulation par un composé à insaturation oléfinique, en particulier le méthylène bis acrylamide. On peut plus particulièrement utiliser un copolymère réticulé acrylamide/chlorure de méthacryloyloxyéthyl triméthylammonium (20/80 en poids) sous forme de dispersion comprenant 50 % en poids dudit copolymère dans de l'huile minérale. Cette dispersion est commercialisée sous le nom de « SALCARE® SC 92 » par la Société CIBA. On peut également utiliser un homopolymère réticulé du chlorure de méthacryloyloxyéthyl triméthylammonium comprenant environ 50 % en poids de l'homopolymère dans de l'huile minérale ou dans un ester liquide. Ces dispersions sont commercialisées sous les noms de « SALCARE® SC 95 » et « SALCARE® SC 96 » par la Société CIBA.
(2) les cyclopolymères d'alkyl diallyl amine ou de dialkyl diallyl ammonium tels que les homopolymères ou copolymères comportant comme constituant principal de la chaîne des motifs répondant aux formules (VII) ou (VIII) : 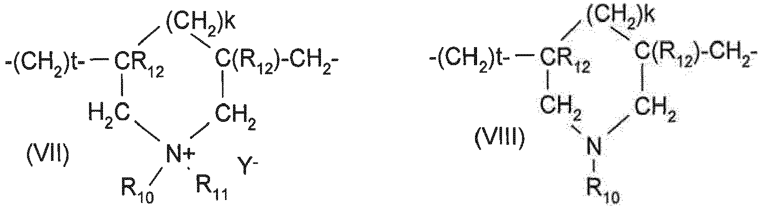 formules dans lesquelles k et t sont égaux à 0 ou 1, la somme k + t étant égale à 1 ; R₁₂ désigne un atome d'hydrogène ou un radical méthyle ; R10 et R11, indépendamment l'un de l'autre, désignent un groupement alkyle ayant de 1 à 6 atomes de carbone, un groupement hydroxyalkyle dans lequel le groupement alkyle a de préférence 1 à 5 atomes de carbone, un groupement amidoalkyle inférieur (C₁-C₄) ou R₁₀ et R₁₁ peuvent désigner conjointement avec l'atome d'azote auquel ils sont rattachés, des groupement hétérocycliques, tels que pipéridinyle ou morpholinyle ; Y-est un anion tel que bromure, chlorure, acétate, borate, citrate, tartrate, bisulfate, bisulfite, sulfate, phosphate. Ces polymères sont notamment décrits dans le brevet français 2.080.759 et dans son certificat d'addition 2.190.406.
   R₁₀ et R₁₁, indépendamment l'un de l'autre, désignent de préférence un groupement alkyle ayant de 1 à 4 atomes de carbone.
   Parmi les polymères définis ci-dessus, on peut citer plus particulièrement les homopolymères de sels (par exemple chlorure) de diméthyldiallylammonium vendus notamment sous la dénomination "MERQUAT 100" par la société NALCO (et ses homologues de faibles masses molaires moyenne en poids) et les copolymères de chlorure de diallyldiméthylammonium et d'acrylamide.
(3) Les copolymères quaternaires de vinyllactame (vinylpyrrolidone et/ou vinylcaprolactame) et de vinylimidazole
(4) le polymère de diammonium quaternaire contenant des motifs récurrents répondant à la formule : 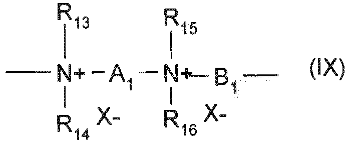 formule (IX) dans laquelle :
   R₁₃, R₁₄, R₁₅ et R₁₆, identiques ou différents, représentent des radicaux aliphatiques, alicycliques, ou arylaliphatiques contenant de 1 à 20 atomes de carbone ou des radicaux hydroxyalkyl aliphatiques inférieurs en C₁-C₆, ou bien R₁₃, R₁₄, R₁₅ et R₁₆, ensemble ou séparément, constituent avec les atomes d'azote auxquels ils sont rattachés des hétérocycles comprenant éventuellement un second hétéroatome autre que l'azote ou bien R₁₃, R₁₄, R₁₅ et R₁₆ représentent un radical alkyle en C₁-C₆ linéaire ou ramifié substitué par un groupement nitrile, ester, acyle, amide ou -CO-O-R₁₇-D ou - CO-NH-R₁₇- doù R₁₇ est un alkylène et D un groupement ammonium quaternaire ;
   A₁ et B₁ représentent des groupements polyméthyléniques comprenant de 2 à 20 atomes de carbone pouvant être linéaires ou ramifiés, saturés ou insaturés, et pouvant comprendre, liés à ou intercalés dans la chaîne principale, un ou plusieurs cycles aromatiques, ou un ou plusieurs atomes d'oxygène, de soufre ou des groupements sulfoxyde, sulfone, disulfure, amino, alkylamino, hydroxyle, ammonium quaternaire, uréido, amide ou ester, et
   X- désigne un anion dérivé d'un acide minéral ou organique;
   A₁, R₁₃ et R₁₅ peuvent former avec les deux atomes d'azote auxquels ils sont rattachés un cycle pipérazinique ; en outre si A₁ désigne un radical alkylène ou hydroxyalkylène linéaire ou ramifié, saturé ou insaturé, B₁ peut également désigner un groupement (CH₂)ₙ-CO-D-OC-(CH₂)ₚ- dans lequel
   n et p, identiques ou différents, sont des nombres entiers variant de 2 à 20 environ
   D désigne :
      a) un reste de glycol de formule : -O-Z-O-, où Z désigne un radical hydrocarboné linéaire ou ramifié ou un groupement répondant à l'une des formules suivantes :

         -(CH₂-CH₂-O)ₓ -CH₂-CH₂-

         -[CH₂-CH(CH₃)-O]_{y}-CH₂-CH(CH₃)-

         où x et y désignent un nombre entier de 1 à 4, représentant un degré de polymérisation défini et unique ou un nombre quelconque de 1 à 4 représentant un degré de polymérisation moyen ;
      b) un reste de diamine bis-secondaire tel qu'un dérivé de pipérazine ;
      c) un reste de diamine bis-primaire de formule : -NH-Y-NH-, où Y désigne un radical hydrocarboné linéaire ou ramifié, ou bien le radical bivalent

         -CH₂-CH₂-S-S-CH₂-CH₂- ;
      d) un groupement uréylène de formule : -NH-CO-NH-.

   De préférence, X- est un anion tel que le chlorure ou le bromure.
   Ces polymères ont une masse moléculaire moyenne en nombre généralement comprise entre 1000 et 100000.
   Des polymères de ce type sont notamment décrits dans les brevets français 2.320.330, 2.270.846, 2.316.271, 2.336.434 et 2.413.907 et les brevets US 2.273.780, 2.375.853, 2.388.614, 2.454.547, 3.206.462, 2.261.002, 2.271.378, 3.874.870, 4.001.432, 3.929.990, 3.966.904, 4.005.193, 4.025.617, 4.025.627, 4.025.653, 4.026.945 et 4.027.020.
   On peut utiliser plus particulièrement les polymères qui sont essentiellement constitués de motifs récurrents répondant à la formule : 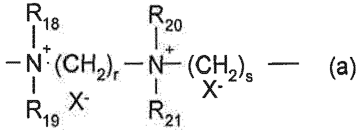 dans laquelle R₁₈, R₁₉, R₂₀ et R₂₁, identiques ou différents, désignent un radical alkyle ou hydroxyalkyle ayant de 1 à 4 atomes de carbone environ, r et s sont des nombres entiers variant de 2 à 20 environ et, X- est un anion dérivé d'un acide minéral ou organique.
   Un composé de formule (a) particulièrement préféré est celui pour lequel R₁₈, R₁₉, R₂₀ et R₂₁, représentent un radical méthyle et r = 3, s = 6 et X = Cl, dénommé Hexadimethrine chloride selon la nomenclature INCI (CTFA).
(5) les polymères de polyammonium quaternaires constitués de motifs de formule (X): 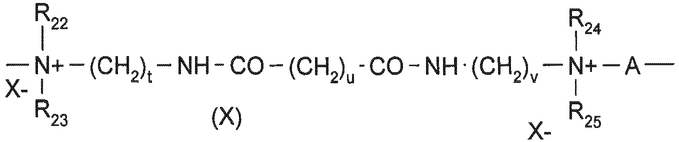 formule dans laquelle :
   R₂₂, R₂₃, R₂₄ et R₂₅, identiques ou différents, représentent un atome d'hydrogène ou un radical méthyle, éthyle, propyle, β-hydroxyéthyle, β-hydroxypropyle ou -CH₂CH₂(OCH₂CH₂)ₚOH,
   où p est égal à 0 ou à un nombre entier compris entre 1 et 6, sous réserve que R₂₂, R₂₃, R₂₄ et R₂₅ ne représentent pas simultanément un atome d'hydrogène,
   t et u, identiques ou différents, sont des nombres entiers compris entre 1 et 6,
   v est égal à 0 ou à un nombre entier compris entre 1 et 34,
   X- désigne un anion tel qu'un halogènure,
   A désigne un radical d'un dihalogénure ou représente de préférence -CH2-CH2-O-CH2-CH2-.

De tels composés sont notamment décrits dans la demande de brevet EP-A-122 324.

On peut par exemple citer parmi ceux-ci, les produits "Mirapol® A 15", "Mirapol® AD 1 ", "Mirapol® AZ1" et "Mirapol® 175" vendus par la société Miranol.

D'autres polymères cationiques utilisables dans le cadre de l'invention sont des polyalkylèneimines, en particulier des polyéthylèneimines, des polymères contenant des motifs vinylpyridine ou vinylpyridinium, des condensats de polyamines et d'épichlorhydrine, des polyuréylènes quaternaires et les dérivés de la chitine.

Parmi tous les polymères cationiques susceptibles d'être utilisés dans le cadre de la présente invention, on préfère mettre en oeuvre les cyclopolymères cationiques, en particulier les homopolymères de chlorure de diméthyldiallylammonium, vendus sous les dénominations « MERQUAT 100 » par la société NALCO (et ses homologues de faibles masses molaires moyenne en poids), les polyéthylèneimines et leurs mélanges.

Selon l'invention, le ou les polymères cationiques ayant une densité cationique supérieure à 4meq/g peuvent représenter de 0,001 % à 10 % en poids, de préférence de 0,005 % à 5 % en poids, et encore plus préférentiellement de 0,01 % à 3 % en poids, du poids total de la composition finale.

Selon un autre mode de réalisation préféré la composition de coloration peut aussi contenir en outre un ou plusieurs tensioactifs oxyalkylénés ou glycérolés non ioniques.

Par tensioactif oxyalkylénés ou glycérolé on entend au sens de la présente invention un composé comportant une ou plusieurs chaînes hydrocarbonée comportant au moins 6 atomes de carbone et au moins un groupement de structure

-CH₂(C(H)ₜ(CH₂R₁)ₙ)_{q}-CH₂ₚ-O-

Avec n ou pou q désignant indépendamment l'un de l'autre 0 ou 1
t désigne 1 ou 2
Et R1 désignant un atome d'hydrogène ou un radical hydroxy.

Ces groupements peuvent être dits oxyéthyléné (q=0, p=1), oxypropyléné(q=1, n=0 t=2 p=1 ou q=1 t= 1 n=1 R1=H) ou glycérolé((q=1, n=0 t=2 p=1 ou q=1 t= 1 n=1 R1=OH)

Plus particulièrement, le tensioactif oxyalkyléné ou glycérolé non ionique est choisi parmi :
les alcools gras oxyalkylénés ou glycérolés ;
les alkylphénols dont la chaîne alkyle est en C8-C18, oxyalkylénés;
les amides gras oxyalkylénés ou glycérolés ;
les huiles végétales oxyalkylénées ;
les esters d'acides C6-C30 du sorbitan oxyalkylénés ;
les esters d'acides gras du sucrose oxyalkylénés ;
les esters d'acides gras du polyéthylèneglycol ;
les copolymères d'oxyde d'éthylène et de propylène ;
leurs mélanges.

Plus particulièrement, le nombre moyen de motifs oxyalkylénés est avantageusement compris entre 2 et 150 motifs. De préférence, il s'agit de motifs oxyéthylénés, oxypropylénés ou leurs mélanges.

En ce qui concerne les tensioactifs glycérolés, ils comportent de préférence en moyenne 1 à 20 groupements glycérol et en particulier 1,5 à 5.

Conformément à un mode de réalisation particulièrement avantageux de l'invention, la composition comprend au moins un tensioactif non ionique choisi parmi les alcools en C₆-C₃₀ oxyalkylénés ou glycérolés

Selon le mode de réalisation particulier de l'invention décrit ci-dessus, la teneur totale en tensioactifs non ioniques oxyalkylénés ou glycérolés représente de 0,01% à 50% en poids par rapport au poids de la composition, de préférence de 0,1 à 30 % en poids par rapport au poids de la composition, mieux de 0,1 à 20% et encore mieux de 0.1 à 10% en poids par rapport au poids de la composition.

La composition de coloration peut en outre contenir un ou plusieurs colorant(s) direct(s) pouvant notamment être choisi(s) parmi les colorants nitrés de la série benzénique, les colorants directs azoïques, les colorants directs méthiniques, et leurs sels d'addition. Ces colorants directs peuvent être de nature non- ionique, anionique ou cationique.

La composition de coloration peut comprendre en outre un ou plusieurs agent(s) oxydant(s).

Un tel agent oxydant est choisi de préférence dans le groupe formé par le peroxyde d'hydrogène, le peroxyde d'urée, les bromates ou ferricyanures de métaux alcalins, les persels tels que les perborates et les persulfates. On peut également utiliser à titre d'agent oxydant une ou plusieurs enzyme(s) d'oxydo-réduction telles que les laccases, les peroxydases et les oxydoréductases à 2 électrons (telles que l'uricase), éventuellement en présence de leur donneur ou cofacteur respectif.

L'utilisation du peroxyde d'hydrogène est particulièrement préférée. Cet agent oxydant est avantageusement constitué par une solution d'eau oxygénée dont le titre peut varier, plus particulièrement, d'environ 1 à 40 volumes, et encore plus préférentiellement d'environ 5 à 40 volumes.

Le milieu approprié pour la coloration appelé aussi support de teinture est un milieu cosmétique comprenant généralement de l'eau ou un mélange d'eau et d'un ou plusieurs solvant(s) organique(s) acceptable(s) sur le plan cosmétique.

A titre d'exemples de solvants organiques, on peut notamment citer les solvants comme les alcools tels que l'alcool éthylique, l'alcool isopropylique, l'alcool benzylique, et l'alcool phényléthylique, ou les glycols ou éthers de glycol tels que, par exemple, les éthers monométhylique, monoéthylique et monobutylique d'éthylèneglycol, le propylèneglycol ou ses éthers tels que, par exemple, le monométhyléther de propylèneglycol, le butylèneglycol, l'hexylène glycol, le dipropylèneglycol ainsi que les alkyléthers de diéthylèneglycol comme par exemple, le monoéthyléther ou le monobutyléther du diéthylèneglycol. Les solvants peuvent alors être présents dans des concentrations comprises entre environ 0,01 à 35% en poids et, de préférence, entre environ 0,1 et 25% en poids par rapport au poids total de la composition.

La composition de coloration peut contenir en outre un ou plusieurs adjuvant(s) utilisé(s) classiquement dans les compositions pour la teinture des cheveux.

Par "adjuvant", on entend un additif, différent des composés précités.

A titre d'exemples d'adjuvants utilisables, on peut citer les agents tensio-actifs anioniques, cationiques, non-ioniques autres de ceux décrits précédemment, amphotères, zwittérioniques ou leurs mélanges ; les polymères anioniques, cationiques autres de ceux décrits précédemment, non-ioniques, amphotères, zwittérioniques, , ou leurs mélanges ; les agents épaississants minéraux ou organiques, et en particulier les épaississants associatifs polymériques anioniques, cationiques, non- ioniques et amphotères ; les agents antioxydants ou réducteurs ; les agents de pénétration ; les agents séquestrants ; les parfums ; les tampons ; les agents dispersants ; les agents conditionneurs tels que par exemple les silicones volatiles ou non volatiles, modifiées ou non modifiées différentes des composés organiques du silicium de l'invention ; les agents filmogènes ; les céramides ; les agents conservateurs ; les agents opacifiants ; et les agents anti-statique.

Les adjuvants ci-dessus sont en général présents en quantité comprise pour chacun d'eux entre 0,01 et 20 % en poids par rapport au poids de la composition tinctoriale.

Bien entendu, l'homme de l'art veillera à choisir le(s) éventuel(s) adjuvant(s) mentionné(s) ci-avant(s), de manière telle que les propriétés avantageuses attachées intrinsèquement aux compositions selon l'invention ne soient pas, ou substantiellement pas, altérées par l'adjonction (les adjonctions) envisagée(s).

Le pH de la composition de coloration est généralement compris entre 3 et 12 environ, de préférence entre 5 et 11 environ. Il peut être ajusté à la valeur désirée au moyen d'agents acidifiants ou alcalinisants habituellement utilisés en teinture des cheveux ou bien encore à l'aide de systèmes tampons classiques.

Parmi les agents alcalinisants, on peut citer, à titre d'exemple, l'ammoniaque, les carbonates alcalins, les alcanolamines telles que les mono-, di- et triéthanolamines ainsi que leurs dérivés, les hydroxyalkylamines et les éthylènediamines oxyéthylénées et/ou oxypropylénées, les hydroxydes de sodium ou de potassium et les composés de formule (XI) suivante : 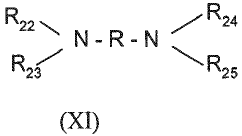 dans laquelle :
▪ R est un reste propylène éventuellement substitué par un groupement hydroxyle ou un radical alkyle en C₁-C₄ ;
▪ R₂₂, R₂₃, R₂₄ et R₂₅, identiques ou différents, représentent un atome d'hydrogène, un radical alkyle en C₁-C₄ ou hydroxyalkyle en C₁-C₄.

Parmi les agents acidifiants, on peut citer, à titre d'exemple, des acides minéraux ou organiques comme l'acide chlorhydrique, l'acide ortho-phosphorique, des acides carboxyliques comme l'acide tartrique, l'acide citrique, l'acide lactique, ou des acides sulfoniques.

La composition de coloration peut se présenter sous des formes diverses, telles que sous forme de liquides, de crèmes, de gels, ou sous toute autre forme appropriée pour réaliser une teinture des cheveux.

Le procédé de la présente invention est un procédé dans lequel on applique sur les fibres la composition de coloration sans oxydant telle que définie précédemment, et on révèle la couleur à l'aide d'un ou plusieurs agent(s) oxydant(s). La couleur peut être révélée à pH acide, neutre ou alcalin et l'agent (les agents) oxydant(s) peu(ven)t être ajouté(s) à la composition de coloration juste au moment de l'emploi ou il(s) peu(ven)t être mis en oeuvre à partir d'une composition oxydante le(s) contenant, appliquée simultanément ou séquentiellement à la composition de l'invention. De préférence, cette coloration est révélée à pH neutre.

Selon un mode de réalisation particulier, la composition de coloration sans oxydant est mélangée, de préférence au moment de l'emploi, à une composition contenant, dans un milieu approprié pour la teinture, un ou plusieurs agent(s) oxydant(s). Le mélange obtenu est ensuite appliqué sur les cheveux. Après un temps de pose variant généralement de 1 à 60 minutes environ, de préférence 5 à 45 minutes environ, les cheveux sont rincés, éventuellement lavés au shampooing et rincés à nouveau Ils peuvent être éventuellement séchés.

Le procédé de coloration de l'invention comprend une étape de post traitement qui consiste à appliquer sur les cheveux une composition comprenant un ou plusieurs composés organiques du silicium tels que définis ci-avant. La composition comprenant les composés organiques du silicium ainsi appliquée peuvent être rincée ou non après un éventuel temps de pose. Les cheveux peuvent être ensuite séchés

De préférence, le temps de pose ce cette composition comprenant le ou les composés organiques du silicium est compris entre quelques secondes et 60 minutes, de préférence entre 30 secondes et 15 minutes et encore plus préférentiellement entre 1 minute et 5 minutes.

De préférence, le procédé de l'invention comprend une étape de rinçage après l'étape de post traitement avec la composition comprenant le ou les composés organiques du silicium décrits auparavant.

L'invention a aussi pour objet un dispositif à plusieurs compartiments ou "kit" de teinture dans lequel un premier compartiment contient la composition de coloration telle que définie ci-dessus à l'exception de l'agent (les agents) oxydant(s), un deuxième compartiment contient un ou plusieurs agent(s) oxydant(s) et un troisième compartiment comprenant un ou plusieurs composés organique du silicium tels que définis précédemment. Ce dispositif peut être équipé d'un moyen permettant de délivrer sur les cheveux le mélange souhaité, tel que les dispositifs décrits dans le brevet FR-A-2 586 913 au nom de la Demanderesse.

La présente invention a également pour objet l'utilisation d'une composition comprenant un ou plusieurs composés organiquse du silicium tels que définis précédemment pour le traitement des cheveux teints par une composition de coloration comprenant un ou plusieurs précurseurs de colorant.

Les exemples suivants servent à illustrer la présente invention.

### EXEMPLE 1

Les compositions suivantes sont réalisées (quantité en gramme de matière active) : Composition aqueuse contenant au moins un composé organique du silicium :

| | |
|---|---|
| Acide lactique | 10,8 |
| Hydroxyéthylcellulose (Natrosol 250 HHR d'AQUALON) | 0,4 |
| 3-Aminopropyltriéthoxysilane Dow Corning Z-6011 Silane | 30 |
| Eau | Qsp 100 |

Composition contenant des colorants d'oxydation et un agent alcalin :

| | |
|---|---|
| Acide oleique | 2,7 |
| Hydroxyde d'ammonium | 2,22(exprimée en NH3) |
| Pentasodium pentetate | 0,8 |
| Monoéthanolamine | 0,63 |
| 2-Oleamido-1,3-Octadecanediol | 0,01 |
| 2,5-Diaminotoluène | 0,7623 |
| Resorcinol | 0,66 |
| m-Aminophenol | 0,14 |
| 2,4-Diaminophenoxyethanol 2HCl | 0,02 |
| Alcool cetearylique | 16,2 |
| Alcool oleique | 2,7 |
| Chlorure d'Hexadimethrine(Mexomere PO de CHIMEX) | 3 |
| Oleth-30 | 3,6 |
| Metabisulfite de sodium | 0,71 |
| Parfum | 0,5 |
| Eau | Qsp 100 |

Composition contenant de l'eau oxygénée :

| | |
|---|---|
| Trideceth Carboxamide Mea | 0,85 |
| Stannate de sodium | 0,04 |
| Pentasodium pentetate | 0,06 |
| Glycerine | 0,5 |
| Alcool cétéarylique | 2,28 |
| Ceteareth-25 | 0,57 |
| Peroxyde d'hydrogène | 6 |
| Pyrophosphate tetrasodique | 0,02 |
| Acide phosphorique | Qs pH2 |
| Eau | Qsp 100 |

La composition contenant des colorants d'oxydation est diluée extemporanément avec 1 fois et demi son poids de la composition comprenant l'agent oxydant.

Le mélange ainsi réalisé est appliqué sur des cheveux châtains fins. Après 30 minutes de pause, les cheveux sont rincés, lavés avec un shampooing standard.

On applique alors sur ces cheveux en post traitement de la composition de coloration, la composition comprenant le composé 3-Aminopropyltriéthoxysilane.

Après 5 minutes de pause, la composition est rincée.

Après éclaircissement et coloration, on obtient en final des cheveux teints dans une nuance chatain clair. La chevelure présente des propriétés coiffantes marquées avec beaucoup de volume et est plus corporisée.

## Revendications

1. Procédé de coloration des cheveux qui comprend, dans l'ordre, une étape de coloration par application d'une composition comprenant un ou plusieurs précurseurs de colorant, une étape de rinçage et une étape de post-traitement des cheveux par application d'une composition aqueuse comprenant au moins 30 % en poids d'eau et au moins 20 % en poids d'un ou plusieurs composés organiques du silicium choisis parmi les composés de formule (III) : 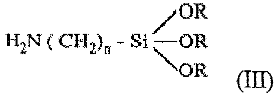 dans laquelle les radicaux R, identiques ou différents, sont choisis parmi les radicaux alkyle en C₁-C₆ et n est un nombre entier de 1 à 6, de préférence de 2 à 4.

2. Procédé selon l'une quelconque des revendications précédentes, dans lequel le composé organique du silicium est le 3-aminopropyl triéthoxysilane

3. Procédé selon l'une quelconque des revendications précédentes dans lequel le ou les composés organiques du silicium sont présents dans une teneur allant de 20 à 65% en poids, préférentiellement de 30 à 60% du poids total de la composition les contenant.

4. Procédé selon l'une quelconque des revendications précédentes dans lequel l'eau est présente dans une teneur allant de 30 à 78 % en poids, préférentiellement de 40 à 70%.

5. Procédé selon l'une quelconque des revendications précédentes, dans lequel l'étape de coloration est mise en oeuvre à partir d'un précurseur de colorant choisi parmi les bases d'oxydation et les coupleurs.

6. Procédé selon l'une quelconque des revendications précédentes, dans lequel l'étape de coloration est mise en oeuvre à partir de bases d'oxydation choisies parmi les ortho-et para-phénylènediamines, les bases doubles, les ortho- et para-aminophénols, les bases hétérocycliques, ainsi que les sels d'addition de ces composés avec un acide et les coupleurs choisis parmi les méta-aminophénols, les méta-phénylènediamines, les méta-diphénols, les naphtols, les coupleurs hétérocycliques et les sels d'addition de ces composés avec un acide.

7. Procédé selon l'une quelconque des revendications précédentes dans lequel l'étape de coloration est mise en oeuvre avec une composition qui comprend un ou plusieurs polymères cationiques dont la densité de charge cationique est supérieure ou égale à 4.

8. Procédé selon l'une quelconque des revendications précédentes dans lequel l'étape de coloration est mise en oeuvre avec une composition qui comprend un ou plusieurs tensioactifs non ioniques polyoxyéthylène ou glycérolés.

9. Procédé selon l'une quelconque des revendications précédentes, dans lequel l'étape de coloration est mise en oeuvre en présence d'un ou plusieurs agent(s) oxydant(s), l'agent oxydant étant de préférence le peroxyde d'hydrogène.

10. Utilisation d'une composition comprenant un composé organique du silicium tels que définis à l'une quelconque des revendications 1 ou 2 sur les cheveux à la suite d'une composition de coloration comprenant un ou plusieurs précurseurs de colorant.

11. Dispositif à plusieurs compartiments ou "kit" de teinture dans lequel un premier compartiment contient une composition de coloration telle que définie à l'une quelconque des revendications 1 et 5 à 8, un deuxième compartiment contient un ou plusieurs agent(s) oxydant(s) et un troisième compartiment comprenant un ou plusieurs composés organique du silicium tels que définis à l'une quelconque des revendications 1 ou 2.

## Patentansprüche

1. Haarfärbeverfahren, umfassend in der angegebenen Reihenfolge einen Schritt des Färbens durch Aufbringen einer Zusammensetzung, die eine oder mehrere Farbstoffvorstufen umfasst, einen Schritt des Spülens und einen Schritt des Nachbehandelns der Haare durch Aufbringen einer wässrigen Zusammensetzung, die mindestens 30 Gew.-% Wasser und mindestens 20 Gew.-% einer oder mehrerer organischer Siliciumverbindungen, die aus den Verbindungen der Formel (III): 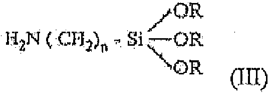 in der die Reste R gleich oder verschieden sind und aus C₁-C₆-Alkylresten ausgewählt sind und n für eine ganze Zahl von 1 bis 6, vorzugsweise 2 bis 4, steht, ausgewählt sind, umfasst.

2. Verfahren nach einem der vorhergehenden Ansprüche, bei dem es sich bei der organischen Silicium-verbindung um 3-Aminopropyltriethoxysilan handelt.

3. Verfahren nach einem der vorhergehenden Ansprüche, bei dem die organische Siliciumverbindung bzw. die organischen Siliciumverbindungen in einer Menge im Bereich von 20 bis 65 Gew.%, vorzugsweise 30 bis 60%, bezogen auf das Gesamtgewicht der sie enthaltenden Zusammensetzung, vorliegen.

4. Verfahren nach einem der vorhergehenden Ansprüche, bei dem das Wasser in einer Menge im Bereich von 30 bis 78 Gew.-%, vorzugsweise 40 bis 70%, vorliegt.

5. Verfahren nach einem der vorhergehenden Ansprüche, bei dem der Färbeschritt mit einer Farbstoffvorstufe, die aus Oxidationsbasen und Kupplern ausgewählt ist, durchgeführt wird.

6. Verfahren nach einem der vorhergehenden Ansprüche, bei dem der Färbeschritt mit Oxidationsbasen, die aus ortho- und para-Phenylendiaminen, Doppelbasen, ortho- und para-Aminophenolen, heterocyclischen Basen sowie Säureadditionssalzen dieser Verbindungen ausgewählt sind und die Kupplern, die aus meta-Aminophenolen, meta-Phenylendiaminen, meta-Diphenolen, Naphtholen, heterocyclischen Kupplern und Säureadditionssalzen dieser Verbindungen ausgewählt sind, durchgeführt wird.

7. Verfahren nach einem der vorhergehenden Ansprüche, bei dem der Färbeschritt mit einer Zusammensetzung, die ein oder mehrere kationische Polymere mit einer Kationenladungsdichte größer gleich 4 umfasst, durchgeführt wird.

8. Verfahren nach einem der vorhergehenden Ansprüche, bei dem der Färbeschritt mit einer Zusammensetzung, die ein oder mehrere Polyoxyethylen oder glycerinierte nichtionische Tenside umfasst, durchgeführt wird.

9. Verfahren nach einem der vorhergehenden Ansprüche, bei dem der Färbeschritt in Gegenwart eines oder mehrerer Oxidationsmittel durchgeführt wird, wobei es sich bei dem Oxidationsmittel vorzugsweise um Wasserstoffperoxid handelt.

10. Verwendung einer Zusammensetzung, die eine organische Siliciumverbindung gemäß einem der Ansprüche 1 oder 2 umfasst, auf den Haaren nach einer Färbezusammensetzung, die eine oder mehrere Farbstoffvorstufen umfasst.

11. Vorrichtung mit mehreren Kompartimenten oder "Kit" zum Färben, wobei ein erstes Kompartiment eine Färbezusammensetzung gemäß einem der Ansprüche 1 und 5 bis 8 enthält, ein zweites Kompartiment ein oder mehrere Oxidationsmittel enthält und ein drittes Kompartiment umfassend eine oder mehrere organische Siliciumverbindungen gemäß einem der Ansprüche 1 oder 2.

## Claims

1. Method for dyeing the hair which comprises, in order, a stage of dyeing by application of a composition comprising one or more dye precursors, a stage of rinsing and a stage of posttreatment of the hair by application of an aqueous composition comprising at least 30% by weight of water and at least 20% by weight of one or more organic silicon compounds chosen from the compounds of formula (III): 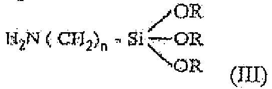 in which the R radicals, which are identical or different, are chosen from C₁-C₆ alkyl radicals and n is an integer from 1 to 6, preferably from 2 to 4.

2. Method according to either one of the preceding claims, in which the organic silicon compound is (3-aminopropyl)triethoxysilane.

3. Method according to any one of the preceding claims, in which the organic silicon compound or compounds are present in a content ranging from 20 to 65% by weight, preferably from 30 to 60%, of the total weight of the composition comprising them.

4. Method according to any one of the preceding claims, in which the water is present in a content ranging from 30 to 78% by weight, preferably from 40 to 70%.

5. Method according to any one of the preceding claims, in which the dyeing stage is carried out starting from a dye precursor chosen from oxidation bases and couplers.

6. Method according to any one of the preceding claims, in which the dyeing stage is carried out starting from oxidation bases chosen from ortho- and para-phenylenediamines, double bases, ortho- and paraaminophenols, heterocyclic bases and the addition salts of these compounds with an acid and the couplers chosen from meta-aminophenols, meta-phenylenediamines, metadiphenols, napththols, heterocyclic couplers and the addiction salts of these compounds with an acid.

7. Method according to any one of the preceding claims, in which the dyeing stage is carried out with a composition which comprises one or more cationic polymers, the cationic charge density of which is greater than or equal to 4.

8. Method according to any one of the preceding claims, in which the dyeing stage is carried out with a composition which comprises one or more polyoxyethylene or glycerolated nonionic surfactants.

9. Method according to any one of the preceding claims, in which the dyeing stage is carried out in the presence of one or more oxidizing agent(s), the oxidizing agent preferably being hydrogen peroxyde.

10. Use of a composition comprising an organic silicon compound as defined in any one of Claims 1 or 2 on the hair following a dyeing composition comprising one or more dye precursors.

11. Dyeing kit or multicompartment device in which a first compartment contains a dyeing composition as defined in any one of Claims 1 and 5 to 8, a second compartment contains one or more oxidizing agent(s) and a third compartment comprising one or more organic silicon compounds as defined in any one of Claims 1 or 2.
